# EUROPEAN PATENT APPLICATION

(11) **EP 1 426 060 A1**
(43) Date of publication of application: **09.06.2004**
(21) Application number: 02798047.3
(22) Date of filing: 09.09.2002
(51) Int. Cl.: A61K 45/00, A61K 31/435, A61P 11/06, A61P 17/00, A61P 17/04, A61P 27/00, A61P 27/16, A61P 37/08, A61P 43/00

(54) **REMEDIES FOR ALLERGIC DISEASES**

(30) Priority: 10.09.2001 JP 2001273349
(71) Applicant: ONO PHARMACEUTICAL CO., LTD., Osaka-shi, Osaka 541-8526 (JP)
(72) Inventor: KOMENO, Masaharu, Ono Pharmaceutical Co. Ltd., Sakai-gu n, Fukui 913-00 (JP); KAMANAKA, Yoshihisa, Ono Pharmaceutical Co. Ltd., Mishima-gun, Osaka 618-8585 (JP)
(74) Representative: Henkel, Feiler & Hänzel
(86) International application number: PCT/JP2002/009150
(87) International publication number: WO 2003/022309

(57) **Abstract**

The present invention relates to an agent for the treatment and/or prevention of allergy which comprises, as an active ingredient, an inducible nitrogen monoxide synthase inhibitor, preferably a condensed piperidine compound represented by formula (I), or an acid addition salt thereof or a hydrate thereof: 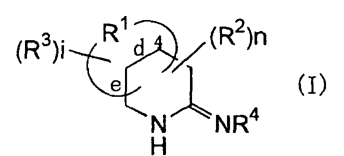 wherein symbols are as defined in the specification.

A therapeutic agent for allergy of the present invention which comprises an inducible nitrogen monoxide synthase inhibitor as an active ingredient is effective at low doses in the treatment of allergy.

## Description

### Field of the Invention

The present invention relates to a therapeutic agent for the treatment of allergy. In more detail, it relates to an agent for the treatment and/or prevention of allergy comprising an inhibitor of inducible nitric oxide synthase, preferably, a condensed piperidine compound represented by formula (I), or an acid addition salt thereof or a hydrate thereof. 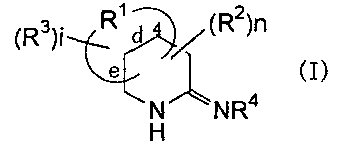 A definition of each symbol is described bellow.

### Background of the Invention

Based on the discovery that macrophages, one of immunocompetent cell, produce large amounts of nitrates, it is discovered that nitric oxide is generated in living organisms [*Proc. Natl. Acad. Sci. USA*, 82, 7738-7742 (1985); *J. Immunol*., 138, 550-565 (1987)]. Also, a substance being released from vascular endothelial cells with relaxant effect was discovered in cardiovascular field, and was named endothelium derived relaxing factor (EDRF). After that, a substance of this EDRF was identified to be NO [*Nature*, 327, 524-526 (1987)].

NO thus become clear to be produced in living body is generated from L-arginine as substrate through the following pathway by nitric oxide synthase (NOS).

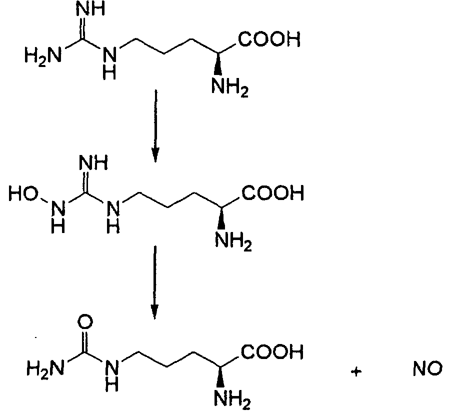

There are at least several isozymes, non-inducible-type NOS (endothelial type and neuronal type) and inducible-type NOS. Endothelial-type NOS exists mainly in vascular endothelial cells and its activity is regulated by intracellular calcium concentration. Neuronal-type NOS exists in central neuron and peripheral neuron, or β-cell of pancreatic island, neuron of the gut, adrenal medulla and nephritic macula densa etc., and its activity is regulated by intracellular calcium concentration as well as endothelial-type NOS.

Endothelial-type NOS and neuronal-type NOS (they are simply referred to as "constitutive NOS or c-NOS") exist in cells constitutively and their enzyme levels do not change by physiologic changes. Inducible-type NOS (simply referred to as "inducible NOS or i-NOS") exists in hepatic parenchymal cells, neutrophils, macrophages, smooth muscle, fibroblasts, nephritic mesangial cells, epithelium of the gut, β-cell of pancreatic island, vascular smooth muscle cells or glia cells etc. It is not found in cells under basal conditions, and is induced by the stimulation of endotoxin and various cytokines.

The action of NO which is produced by NOS is varied, for example, vasodepressor effect, inhibition of platelet aggregation, inhibition of adhesion, inhibition of leukocyte adhesion/chemotaxis, suppression of sympathetic nerve activity, endotoxin shock, low blood pressure caused by endotoxin/cytokine, action as signal transduction molecule among neuron, ischemic injury of brain cells, anti-tumor, bacteriocidal action, autoimmune diseases, insulin-dependent diabetes mellitus, arthritis, tissue damage after transplantation, and transplant rejection etc.

As NOS inhibitor is helpful for the analysis of physiological action of NO in living body and could be used for the treatment of shock or ischemic diseases etc., development of various NOS inhibitors are furthered in recent years.

For example, there are arginine analogues as a substrate competitor, such as N-ω-monomethyl-L-arginine (L-NMMA), N-ω-nitro-L-arginine (L-NNA), N-ω-nitro-L-arginine methylester (L-NAME), N-ω-amino-L-arginine (L-NAA), N-ω-iminoethyl-L-ornithine (L-NIO). Also, there are diphenyleneiodonium (DPI), di-2-thienyliodonium (DTI) and calcineurin etc. as a cofactor competitor.

In ophthalmic region, for example, it is reported that the increase in NO production and vascular permeability was caused by over-expression of NOS in allergic conjunctivitis, and that these were suppressed by L-NAME (*Cornea*, 19(1), 84-91 (Jan. 2000)). Furthermore, it is known that the increase in NO production in acute phase of allergic conjunctivitis causes the formation of edema through prostaglandin E2 production, and that L-NAME suppresses prostaglandin E2 production by inhibiting NO generation (*Prostaglandins*, 52(6), 431-446 (Dec. 1996)).

While compounds shown in formula (I) were applied for a patent as NOS inhibitor (EP870763).

NOS inhibitors were also described in the specification of WO96/35677, WO95/11231, WO96/14844 or WO96/14842.

### Disclosure of the Invention

It is known that a NOS inhibitor is useful for the treatment of various diseases in which NO is involved, as it inhibits NO synthesis in living body. As shown previously, there is information that L-NAME is effective on allergic conjunctivitis, however, the action is weak.

Moreover, it has not been confirmed whether a NOS inhibitor is actually effective on allergosis other than allergic conjunctivitis.

The inventors focused on the compounds shown in formula (I), and as a result of repeating examination wholeheartedly, the inventors first found out that they were effective at low doses in allergic rhinitis model and completed this invention.

That is, the present invention relates to an agent for the treatment and/or prevention of allergy which comprises, as an active ingredient, a condensed piperidine compound represented by formula (I), or an acid addition salt thereof or a hydrate thereof: 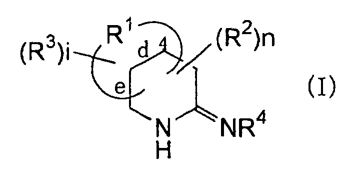
wherein -R¹- represents a 3- or 4-membered carbocyclic ring together with the carbon atom or atoms to which it is bonded, said carbocyclic ring being condensed to side d or e of the piperidine ring or bonded to the 4-position of the piperidine ring through a spiro-union;
R² represents a C₁₋₆ alkyl group;
R³ represents a C₁₋₆ alkyl group, a C₂₋₆ alkenyl group, a C₂₋₆ alkynyl group or a halogen atom;
R⁴ represents a hydrogen atom, an amino-C₁₋₄ alkyl group or a carbocyclic ring-C₁₋₄ alkyl group which may be substituted with an amino-C₁₋₄ alkyl group;
i represents 0 or an integer of 1 to 3;
n represents 0 or an integer of 1 to 3;
and the plural R²' s or R³' s are the same or different.

Among compounds represented by formula (I), a preferred compound is a condensed piperidine compound represented by formula (IA), or a non-toxic salt thereof or a hydrate thereof: 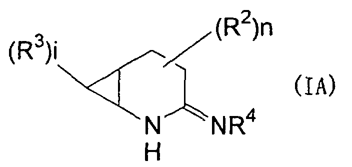 wherein symbols are as defined above.

Among compounds represented by formulas (I) and (IA), a preferred compound is (+)-trans-3-imino-5-methyl-7-chloro-2-azabicyclo[4.1.0] heptane represented by formula (Ia), or a non-toxic salt thereof or a hydrate thereof.

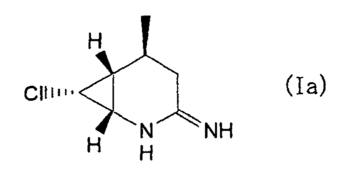

The compound represented by formulas (I), (IA) and (Ia) in the present invention may be administered in the form of its acid addition salt described bellow. It is desirable for the acid addition salt be non-toxic and water-soluble. Suitable acid addition salts include inorganic acid salts (e.g., hydrochloride, hydrobromide, hydroiodide, sulfate, phosphate, nitrate) and organic acid salts (e.g., acetate, lactate, tartrate, oxalate, fumarate, maleate, citrate, benzoate, methanesulfonate, ethanesulfonate, benzenesulfonate, toluenesulfonate, isethionate, glucuronate, gluconate). A hydrochloride salt is preferred.

The compound represented by formulas (I), (IA) and (Ia) or a salt thereof can be converted to its hydrate in a known manner.

An inducible nitrogen monoxide synthase (i-NOS) inhibitor in the present invention means every compound having an inhibitory action on inducible nitrogen monoxide synthase.

Accordingly, not only known inducible nitrogen monoxide synthase inhibitors at the present moment but also possible inducible nitrogen monoxide synthase inhibitors to be newly found from now are included. For example, there are arginine analogues as a substrate competitor, such as N-ω-monomethyl-L-arginine (L-NMMA), N-ω-nitro-L-arginine (L-NNA), N-ω-amino-L-arginine (L-NAA), N-ω-iminoethyl-L-ornithine (L-NIO). Also, there are diphenyleneiodonium (DPI), di-2-thienyliodonium (DTI) and calcineurin etc. as a cofactor competitor, but not limited to these.

NOS inhibitors described in the specification of WO96/35677, WO95/11231, WO96/14844 or WO96/14842 are also useful, and an application of these compounds to allergy is also included in the present invention.

For supplement and/or potentiation of preventive and/or therapeutic response to allergy of the compound represented by general formula (I) in the present invention, there are, for example, antihistamines, release inhibitors of mediator, thromboxane synthetase inhibitors, leukotriene receptor antagonists, steroids, alpha-adrenergic stimulants, xanthine derivatives, anticholinergic drugs, prostaglandins, beta-adrenergic stimulants, phosphodiesterase inhibitors, cannabinoid-2 stimulants.

### Process for preparing the compounds:

The compounds represented by formulas (I), (IA) and (Ia) can be prepared by the process described in EP870763. Pharmacological activity of the compounds:

The compounds represented by formulas (I), (IA) and (Ia) have a potent inhibitory action on inducible nitrogen monoxide synthase, and are effective in allergic rhinitis model as described later.

### Toxicity:

It was confirmed that the compounds were less toxic and quite safe for the use as medicine. For example, Maximum Tolerated Dose of the compound represented by formula (Ia) was 30 mg/ kg by intravenous administration in mice.

### Industrial Applicability

### Application for pharmaceuticals:

The compounds represented by formulas (I), (IA) and (Ia) , or an acid addition salt thereof or a hydrate thereof are useful for the treatment and/or prevention of allergy such as anaphylactic shock, allergic rhinitis, bronchial asthma, urticaria, atopic dermatitis, hay fever and metal allergy.

For the above mentioned usage of the compounds represented by formulas (I), (IA) and (Ia), or an acid addition salt thereof or a hydrate thereof, administration can be carried out in systemic or local, generally peroral or parenteral ways.

The dosage to be administered depends upon age, body weight, symptom, desired therapeutic effect, route of administration, and duration of the treatment etc. In human adults, one dose per person is generally between 1 mg and 1000 mg by oral administration up to several times per day, or between 0.1 mg and 100 mg by parenteral administration (preferably, administration of eye drops) up to several times per day, or continuous administration between 1 and 24 hrs. per day into vein.

As mentioned above, the doses to be used depend upon various conditions. Therefore, there are cases in which doses lower than or greater than the ranges specified above may be used.

The compounds of the present invention may be administered as eye drops, injections, liniments or suppositories etc. for parenteral administration, or as inner solid compositions or inner liquid compositions for oral administration.

Nasal drops for parenteral administration include solutions, suspensions and emulsions, and solid, which are dissolved in solvent when it is used. In such compositions, one or more active compound(s) is/are dissolved in a solvent such as water, higher alcohol and polyhydric alcohol, which are used generally. Such compositions may comprise additional assisting agents for dissolving, isotonic agents, buffer agents, pH regulator, stabilizing agents, preserving agents, detergent, viscosity improver and suspending agents.

Injections for parenteral administration include solutions, suspensions and emulsions, and solid injections, which are dissolved or suspended in solvent when it is used. In such compositions, one or more active compound(s) is/are dissolved, suspended or emulsified in a solvent. Solvents include distilled water for injection, physiological salt solution, plant oil, propylene glycol, polyethylene glycol, alcohol such as ethanol, and mixture thereof etc. Such compositions may comprise additional stabilizing agents, assisting agents for dissolving (glutamic acid, asparaginic acid, POLYSOLBATE80 (resistered trade mark) etc.), suspending agents, emulsifying agents, soothing agent, buffer agents, preserving agents etc. They may be manufactured or prepared by sterilization or by aseptic manipulation in a final process. They may also be manufactured in the form of sterile solid compositions such as freeze-dried compositions, and can be dissolved in sterile water or some other sterile solvent for injection immediately before use.

Other compositions for parenteral administration include liquids for external use, ointments, endermic liniments, aerosols, spray compositions, suppositories and pessaries for vaginal administration etc., which comprise one or more of the active compound(s) and may be prepared by known methods.

Spray compositions may comprise additional substances other than inert diluents generally used: e.g. stabilizing agents such as sodium hydrogen sulfate, buffer agents to give isotonicity, isotonic buffer such as sodium chloride, sodium citrate and citric acid. For preparation of such spray compositions, for example, the methods described in the United States Patent No. 2,868,691 and 3,095,355 may be used.

Examples of Inner solid compositions for oral administration include compressed tablets, pills, capsules, dispersible powders and granules etc. Examples of capsules include hard capsules and soft capsules.

In such inner solid compositions, one or more of the active compound(s) remains intact, or is/are admixed with excipients (lactose, mannitol, glucose, microcrystalline cellulose and starch etc.), connecting agents (hydroxypropyl cellulose, polyvinylpyrrolidone, magnesium metasilicate aluminate, etc.), disintegrating agents (cellulose calcium glycolate etc.), lubricating agents (magnesium stearate etc.), stabilizing agents, assisting agents for dissolving (glutamic acid, asparaginic acid etc.) etc. to prepare pharmaceuticals by known methods. The pharmaceuticals may, if desired, be coated with coating agent (sugar, gelatin, hydroxypropyl cellulose or hydroxypropylmethyl cellulose phthalate etc.), or be coated with two or more films. Further, coating may include capsules of absorbable materials such as gelatin.

Inner liquid compositions for oral administration may contain pharmaceutically acceptable water-agents, suspensions, emulsions, syrups and elixirs etc. In such liquid compositions, one or more of the active compound(s) is/are resolved, suspended or emulsified in inert diluent(s) commonly used in the art (purified water, ethanol or mixture thereof etc.). Such liquid compositions may also comprise wetting agents, suspending agents, emulsifying agent, sweetening agents, flavoring agents, perfuming agents, preserving agents and buffer agents etc.

### Brief Description of the Drawings

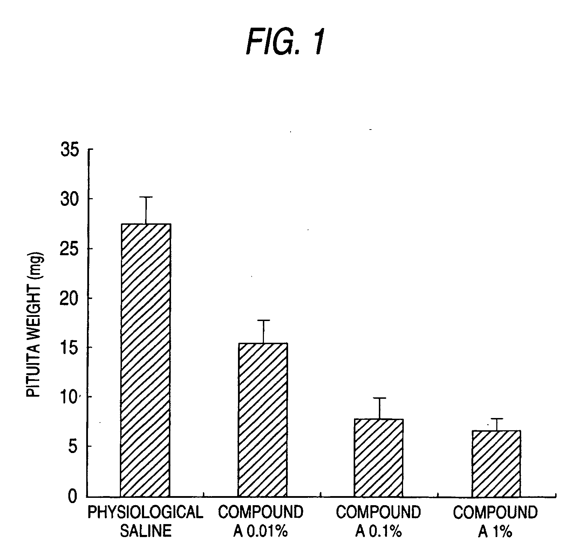

Figure 1 is a graph showing that (+)-trans-3-imino-5-methyl-7-chloro-2-azabicyclo[4.1.0] heptane monohydrochloride salt (compound A), a nitrogen monoxide synthase inhibitor, suppresses pituita weight in dose dependent manner.

### Best Mode for Carrying Out the Invention

The present invention is more specifically explained by means of the following test examples, but is not limited only to these test examples.

### Test Example 1: allergic rhinitis model

### Method:

Allergic rhinitis model was produced by administration of ovalbumin to male Crj Hartley guinea pig (6 weeks old) in the procedure shown in table 1.

**Table 1**

| Day | Dose | administration route |
|---|---|---|
| 0 | 0.5mg/0.5mL | intraperitoneal |
| 2 | 1.0mg/0.5mL | intraperitoneal |
| 22 | 0.1% 40uL | Nasal (both sides) |
| 24 | 0.2% 40uL | Nasal (both sides) |
| 27 | 0.4% 40uL | Nasal (both sides) |
| 31 | 0.5% 40uL | Nasal (both sides) |
| 36 | 0.1% 40uL | Nasal (both sides) |
| 41 | 0.1% 40uL | Nasal (both sides) |

At day 42, guinea pigs were inserted with tube into the trachea under anesthesia and kept the heat using heat pat. Forty micro liter of (+)-trans-3-imino-5-methyl-7-chloro-2-azabicyclo[4.1.0] heptane monohydrochloride salt (referred to as "compound A") or physiological saline was administered nasally into both sides. Ten minutes later, 40 uL of 1 % ovalbumin was administered nasally into both sides. Thirty minutes after ovalbumin administration, moisture in the nose was removed with absorbent cotton. Fifteen minutes later, absorbent cotton of which weight was measured was inserted into nose for 15 minutes. Pituita weight was calculated by the difference in weight between pre and post insertion.

### Results:

Pituita weights in the case that 0.01%, 0.1% and 1.0% solution of compound A, and physiological saline was administered nasally are shown in figure 1 (vertical axis represents mg).

Figure 1 shows that 0.01%, 0.1% and 1.0% solution of compound A noticeably suppresses pituita weights as compared to control (physiological saline), and the degree of suppression is dose dependent.

### Formulation Example 1: Preparation of nasal drops

(+)-trans-3-imino-5-methyl-7-chloro-2-azabicyclo[4.1.0] heptane monohydrochloride salt (100 mg) was dissolved in distilled water (100 ml), and 10 ml of the solution was poured into 50 ml container to obtain 10 nasal drops comprising 10 mg of active ingredient.

## Claims

1. An agent for the treatment of allergy excluding eye diseases which comprises, as an active ingredient, an inducible nitrogen monoxide synthase inhibitor.

2. The agent for the treatment and/or prevention of allergy according to claim 1, wherein the inducible nitrogen monoxide synthase inhibitor is a condensed piperidine compound represented by formula (I), or an acid addition salt thereof or a hydrate thereof: 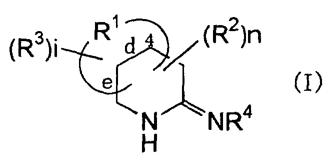
wherein -R¹- represents a 3- or 4-membered carbocyclic ring together with the carbon atom or atoms to which it is bonded, said carbocyclic ring being condensed to side d or e of the piperidine ring or bonded to the 4-position of the piperidine ring through a spiro-union;
R² represents a C₁₋₆ alkyl group;
R³ represents a C₁₋₆ alkyl group, a C₂₋₆ alkenyl group, a C₂₋₆ alkynyl group or a halogen atom;
R⁴ represents a hydrogen atom, an amino-C₁₋₄ alkyl group or a carbocyclic ring-C₁₋₄ alkyl group which may be substituted with an amino-C₁₋₄ alkyl group;
i represents 0 or an integer of 1 to 3;
n represents 0 or an integer of 1 to 3;
and the plural R²'s or R³' s are the same or different.

3. The agent for the treatment and/or prevention of allergy according to claim 2, wherein the inducible nitrogen monoxide synthase inhibitor is a condensed piperidine compound represented by formula (IA), or a non-toxic salt thereof or a hydrate thereof: 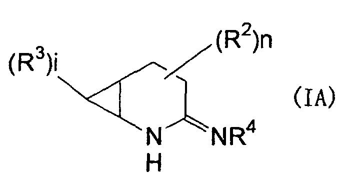 wherein symbols are as defined in claim 1.

4. The agent for the treatment and/or prevention of allergy according to claim 3, wherein the inducible nitrogen monoxide synthase inhibitor is (+)-trans-3-imino-5-methyl-7-chloro-2-azabicyclo[4.1.0] heptane represented by formula (Ia), or a non-toxic salt thereof or a hydrate thereof.

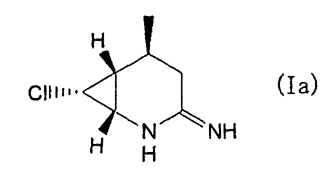

5. The agent for the treatment and/or prevention of allergy according to claim 1, wherein the inducible nitrogen monoxide synthase inhibitor is a compound described in the specification of WO96/35677, WO95/11231, WO96/14844 or WO96/14842.

6. The agent for the treatment and/or prevention of allergy according to any one of claims 1-5, wherein the allergy is anaphylactic shock.

7. The agent for the treatment and/or prevention of allergy according to any one of claims 1-5, wherein the allergy is allergic rhinitis.

8. The agent for the treatment and/or prevention of allergy according to any one of claims 1-5, wherein the allergy is bronchial asthma.

9. The agent for the treatment and/or prevention of allergy according to any one of claims 1-5, wherein the allergy is urticaria.

10. The agent for the treatment and/or prevention of allergy according to any one of claims 1-5, wherein the allergy is atopic dermatitis.

11. The agent for the treatment and/or prevention of allergy according to any one of claims 1-5, wherein the allergy is hay fever.

12. The agent for the treatment and/or prevention of allergy according to any one of claims 1-5, wherein the allergy is metal allergy.
